# EUROPÄISCHE PATENTSCHRIFT

(11) **EP 1 109 837 B1**
(45) Veröffentlichungstag und Bekanntmachung des Hinweises auf die Patenterteilung: **16.11.2005**
(21) Anmeldenummer: 99941584.7
(22) Anmeldetag: 12.08.1999
(51) Int. Cl.: C08B 37/04

(54) **VERFAHREN ZUR GEWINNUNG HOCHGEREINIGTER ALGINATE**
METHOD FOR PRODUCING ULTRA-PURE ALGINATES
PROCEDE POUR OBTENIR DES ALGINATES ULTRAPURS

(30) Priorität: 14.08.1998 DE 19836960
(43) Veröffentlichungstag der Anmeldung: 27.06.2001
(73) Patentinhaber: CellMed AG, 63755 Alzenau (DE)
(72) Erfinder: ZIMMERMANN, Ulrich, D-97295 Waldbrunn (DE); BEHRINGER, Marcus, D-97299 Zell/Main (DE)
(74) Vertreter: Schneider, Michael
(86) Internationale Anmeldenummer: PCT/EP1999/005867
(87) Internationale Veröffentlichungsnummer: WO 2000/009566

(56) Entgegenhaltungen:
- WO-A-93/16111
- WO-A-93/24077
- US-A- 5 459 054
- US-A- 5 622 718
- KLOCK G ET AL: "Biocompatibility of mannuronic acid-rich alginates" BIOMATERIALS,GB,ELSEVIER SCIENCE PUBLISHERS BV., BARKING, Bd. 18, Nr. 10, Seite 707-713 XP004063784 ISSN: 0142-9612

## Beschreibung

Die Erfindung betrifft ein Verfahren zur Gewinnung hochgereinigter Alginate, insbesondere aus Braunalgen, und mit diesem Verfahren hergestellte Alginate mit einem hohen Polymerisationsgrad sowie deren Verwendung.

Alginate besitzen zahlreiche Anwendungen im Bereich der Lebensmitteltechnik (z.B. Askar in "Alimenta", Bd. 21, 1982, S. 165 ff.) und in der Textiltechnik, in zunehmendem Maße jedoch auch in der Pharmazie, Medizin, Biochemie und Biotechnologie. Die nach den bisher bekannten Verfahren aus Algenpflanzen gewonnenen Alginate (Übersicht beispielsweise in D.J. McHugh: "Production and Utilization of Products from Commercial Seaweets" in "FAO Fisheries Technical Papers", Bd. 288, 1987, Chap. 2) sind durch Schwankungen der Zusammensetzung und der Struktur sowie durch Verunreinigungen gekennzeichnet. Dies ergibt sich daraus, daß das Rohalginat aus Biomasse extrahiert wird, die aus Wildpopulationen gewonnen wird. Die insbesondere in Küstengewässern wachsenden Algenpopulationen sind zahlreichen geographischen, saisonalen und stofflichen (Umweltverschmutzung) Einflüssen ausgesetzt. Hinzu kommt, daß bei der Ernte die Algen ggf. gemeinsam mit Fremdstoffen eingesammelt und zur Konservierung bzw. zur Entfärbung einer chemischen Behandlung (z.B. mit Formalin und/oder Hypochlorid) unterzogen werden.

Die bis jetzt verfügbaren Rohalginate sind daher Mischpolymere variabler Struktur mit Verunreinigungen, zu denen insbesondere toxische Chemikalien zählen können. Da in der Lebensmittel- und Textiltechnik vorrangig Interesse an den gelbildenden Eigenschaften der Alginate besteht, wurden Verfahren zur Nachreinigung oder Reinigung der Rohalginate, wie sie im folgenden erläutert werden, erst für die biologischmedizinischen Anwendungen entwickelt.

Verfahren zur Reinigung von Alginaten (wie sie z.B. von den Unternehmen Keltone LV oder Kelco Nutrasweet verfügbar sind) werden beispielsweise in DE-OS 4 204 012, US-A 5 429 821 (bzw. US-A 5 656 468) und in der Publikation von P. De Vos et al. in "Diabetologia" (Bd. 40, 1997, S. 262 ff.) beschrieben. Diese Verfahren besitzen generell die Nachteile eines hohen Energieaufwandes (Anwendungen einer Elektrophorese, Gefriertrocknung oder Zentrifugation, Erhitzung oder Kochen), einer hohen Umweltbelastung (Verwendung von Säuren wie HCl, H₂SO₄, biologisch nicht-abbaubaren Lösungsmitteln wie CHCl₃ oder Schwermetallionen wie beispielsweise Barium, Blei oder Kadmium) und einer Beschränkung des erzielbaren mittleren Molekulargewichts des gereinigten Endmaterials. Weitere Nachteile der herkömmlichen Verfahren ergeben sich im einzelnen aus den folgenden Erläuterungen:

Bei dem aus DE-OS 42 04 012 bekannten Verfahren wird eine Rohalginatlösung einer Behandlung mit einem Komplexbildner, einer Säureextraktion bei hohen Temperaturen (rund 70 °C), einer Waschung, einer Behandlung mit konzentriertem Alkohol (rund 80 %) und einer weiteren Behandlung mit einem Komplexbildner ausgesetzt. Anschließend folgt ein Dialysevorgang und eine Gefriertrocknung (oder Elektrophorese oder Zentrifugation) zur Gewinnung des gereinigten Alginats. Dieses Verfahren ist wegen des hohen Energieaufwands, der großen Anzahl von Verfahrensschritten, dem Einsatz von toxischen Materialien (z.B. Barium als Komplexbildner) und der Beschränkung auf Alginate (< 500 kD) nachteilig. Ein besonderes Problem ist jedoch, daß der Reinigungseffekt dieses Verfahrens nur beschränkt ist.

In DE-OS 42 04 012 werden die gereinigten Alginate zwar als mitogenfreie Substanzen benannt, dies jedoch lediglich unter der Annahme einer Mitogenfreiheit, falls in vorbestimmten Tierversuchen keine Entzündungsreaktionen beobachtet werden, sowie einer Unbedenklichkeit weiterer Komponenten, die im hochgereinigen Alginat verblieben. Es hat sich jedoch gezeigt, daß die am Ende der 80er Jahre entwickelten und in DE-OS 42 04 012 implementierten Tierversuche nicht geeignet sind, eine Mitogenfreiheit nachzuweisen, die den Anforderungen der modernen biomedizinischen Anwendungen, z.B. der Implantationstechnik, erfüllt. So wurden die Tierversuche beim herkömmlichen Reinigungsverfahren mit sogenannten Lewis-Ratten durchgeführt. Inzwischen konnte jedoch durch G. Klöck et al. in "Biomaterials" (Bd. 18, 1997, S. 707ff) und durch P. Gröhn (Dissertation Universität Würzburg, 1998) nachgewiesen werden, daß die Lewis-Ratten eine relativ geringe Empfindlichkeit gegenüber mitogenen Substanzen besitzen. Implantierte Alginat-Kapseln, die bei Lewis-Ratten nach drei Wochen keine Entzündungsreaktionen auslösten, führten beispielsweise bei sogenannten BB-OK-Ratten ("Bio Breeding / Ottawa Karlsburg") zu Entzündungsreaktionen. Daraus ergibt sich, daß die aus DE-OS 42 04 012 bekannten mitogenfreien Substanzen tatsächlich nicht als hochgereinigt betrachtet werden können und wegen verminderter Biokompatibilität bei biomedizinischen Anwendungen nur beschränkt einsetzbar sind.

Bei dem aus US-A 5,429,821 bzw. US-A 5,656,468 bekannten Reinigungsverfahren wird ebenfalls eine Säurefällung durchgeführt. Es sind wiederum Hochtemperaturverfahrensschritte und zur endgültigen Alginatgewinnung eine Zentrifugation, Elektrophorese oder Gefriertrocknung erforderlich. Die nach diesem Verfahren gewonnenen Alginate sind auf Molekulargewichte unterhalb 200 kD beschränkt. Es folgt eine Trocknung bei 80°C, bei der im Mischpolymer Trocknungsartefakte durch Struktur- oder Stoffumsetzungen entstehen können.

Ein besonderes Problem stellt die Beschränkung auf relativ geringe Molekulargewichte dar. So ist beispielsweise aus "Immobilized Enzymes" von J. Chibata (A Halsted Press Book, John Wiley & Sons, 1978) bekannt, daß die Bio-Toxizität von Materialien mit der Zunahme des Molekulargewichts abnimmt.

Schließlich ist auch das oben genannte Verfahren nach P. de Vos et al. durch zahlreiche Verfahrensschritte, einschließlich einer Säurefällung, dem Einsatz toxischer Chemikalien (Chloroform), die Verwendung hochkonzentrierten Ethanols (rund 70%) und die Anwendung einer Zentrifugation, Elektrophorese oder Gefriertrocknung gekennzeichnet.

Aus WO-A-93/24077 sind ebenfalls Beschichtungsmaterialien aus Alginaten bekannt. Das hier vorgestellte Verfahren geht bereits von Alginaten aus, die im Handel erhältlich sind. Dieses Alginatpräparat wird mit einem Komplexbildner behandelt, um die zweiwertigen Metallionen zu entfernen. Die anschließende Behandlung mit Aktivkohle absorbiert die noch vorhandenen Polyphenole mit den damit verbundenen Proteinen und Fucoseeinheiten. Nach der Behandlung mit Aktivkohle wird das Alginat aus der Lösung gefällt, gewaschen und dann filtriert, um weitere Verunreinigungen zu entfernen. Das Molekulargewicht dieser Alginate ist eher im unteren Bereich anzusiedeln, es liegt im Bereich von etwa 2 bis 300 kD.

Mannuronsäure-reiche Alginate sind aus Biomaterials, Band 8, 1997, Seiten 707 bis 713 bekannt. Dieses Alginat wird aus Braunalgen extrahiert und anschließend gereinigt. Das Reinigungsverfahren basiert auf der Extraktion von mit Ba²⁺-Ionen vernetzten Alginatperlen (hergestellt aus kohlebehandeltem Rohalginextrakt) mit schwachen Säuren, Citrat und Ethanol. Anschließend wird dann dialysiert, wonach dann das Alginat mit Ethanol gefällt wird.

WO-A-93/16111 offenbart eine mitogen-freie Substanz, die Mischpolymere von 10 bis 90 Mol% Guluronsäure und aus jeweils auf 100% ergänzend Mannuronsäure aufweist und die ein Molekulargewicht von 10 bis 500 kD besitzen.

US-A-5,459,054 offenbart ein gereinigtes Alginat mit hohem Guluronsäuregehalt, das von Phenol-ähnlichen Verbindungen und Endotoxinen frei ist.

US-A-5,622,718 offenbart Alginate mit einem mittleren Molekulargewicht von höher als 350 kD mit einem bestimmten Verhältnis von Mannuronsäure zu Guluronsäure.

EP-A-366 868 offenbart die Herstellung von Alginaten, bei denen das Verhältnis von Mannuron- zu Guluronsäure zwischen 7 : 3 und 3 : 7 liegt.

Ein weiterer Nachteil sämtlicher Reinigungsverfahren besteht in deren Beschränkung auf die Reinigung kommerziell verfügbarer Rohalginate. Die Verfahren sind nicht auf Frischmaterial oder geerntete Biomasse anwendbar. Außerdem sind die Verfahren aufgrund der umständlichen Verfahrensführung, des Energieaufwands und dem Einsatz toxischer Chemikalien für eine großtechnische Anwendung nicht praktikabel.

Die Aufgabe der Erfindung ist es, ein Verfahren zur Gewinnung hochgereinigter Alginate anzugeben, mit dem die Nachteile herkömmlicher Reinigungsverfahren vermieden werden und die Herstellung eines hochgereinigten Alginats, insbesondere in großtechnischem Maßstab, ermöglicht wird. Die Erfindung betrifft auch ein Alginat, das nach dem erfindungsgemäßen Verfahren hergestellt wurde und das insbesondere gegenüber den herkömmlichen Alginaten ein erhöhtes Molekulargewicht bzw. eine erhöhte Viskosität hat.

Diese Aufgabe wird durch ein Verfahren bzw. eine Alginatzusammensetzung mit den Merkmalen gemäß den Patentansprüchen 1 bzw. 14 gelöst. Vorteilhafte Ausführungsformen und Verwendungen der Erfindung ergeben sich aus den abhängigen Ansprüchen.

Unter einem hochgereinigten Alginat wird hier eine reproduzierbar herstellbare Alginatzusammensetzung verstanden, die vorbestimmte Molekulargewicht- und/oder Viskositätsparameter besitzt und eine hohe Sterilität, Reinheit und Biokompatibilität aufweist. Letzteres Merkmal bezieht sich beispielsweise darauf, daß mit erfindungsgemäß hochgereinigten Alginaten in autoimmundiabetischen BB/OK-Ratten auch nach mehrwöchiger Implantation keine oder eine vernachlässigbar geringe Fremdkörperreaktion ausgelöst wird.

Im Unterschied zu den herkömmlichen Reinigungsverfahren, die sämtlich an kommerziell verfügbare Rohalginate angepaßt sind, die jedoch Verschnitte oder Mischungen aus verschiedenen Algenmaterialien unter Einschluß von tierischen oder anderen Fremdmaterialien darstellen und somit grundsätzlich nicht hochgereinigtes Alginat ergeben können, wird erfindungsgemäß eine Alginatherstellung oder -gewinnung angegeben, die vorzugsweise von sauberem Algenfrischmaterial oder getrocknetem Algenmaterial als Ausgangsstoff ausgeht. Es ist insbesondere vorgesehen, daß zunächst das Algenmaterial in Gegenwart von Komplexbildnern behandelt wird, worauf mit einem Bindemittel in Form eines Granulats oder einem vergleichbaren porösen Material Zellbestandteile und Partikel sedimentiert werden. Nach einer Filtration erfolgt ein Fällungsschritt, vorzugsweise unter gleichzeitigem Einblasen eines Trägergases, unter dessen Wirkung das ausgefällte Alginat auf der Lösung aufschwimmt. Dieses Aufschäumen (Floatieren) ist nicht zwingend erforderlich. Ausgefälltes Alginat kann auch anderweitig aus der Lösung getrennt werden (z.B. durch einen Dekantiervorgang). Das derart gewonnene Alginat kann von der Lösungsoberfläche abgenommen oder als Rückstand nach dem Dekantieren aufgenommen und an Luft oder mit einer Filterpresse entwässsert werden. Je nach Anwendungsfall wird dieser Reinigungsvorgang bzw. Teilschritte dieses Reinigungsvorgangs einmalig oder mehrmals durchgeführt. Nach der letzten Reinigung erfolgt ein abschließendes Waschen und Lufttrocknen.

Diese Verfahrensweise besitzt die folgenden Vorteile. Bei der Alginatgewinnung wird vollständig auf toxische Chemikalien und Hochtemperaturbedingungen verzichtet. Die einzelnen Verfahrensschritte basieren auf an sich bekannten und einfach beherrschbaren Techniken, deren erfindungsgemäße Kombination besonders vorteilhaft in Bezug auf die Verfahrenskosten, den Materialaufwand und die Prozeßgeschwindigkeit ist. Es wird erstmalig im Gegensatz zu allen herkömmlichen Reinigungsversuchen eine Biokompatibilität des gewonnenen Alginatmaterials erreicht, so daß sich dessen Anwendungsgebiet erheblich, insbesondere in der Biologie und Medizin, erweitert. Das erfindungsgemäß hergestellte, biokompatible Produkt basiert ausschließlich auf Alginat. Das Alginat ist frei von Zusatzstoffen, wie z. B. Immunsuppressiva oder immunstimulierenden Substanzen oder Phenolen oder Phenol-ähnlichen Verbindungen, und kann in diesem Zustand unmittelbar angewendet werden.

Die Verfahrensschritte können ohne Probleme in verhältnismäßig kleinem Maßstab, z.B. am Ort der Algenernte, oder auch großtechnisch durchgeführt werden. Durch gezielte Steuerung der Fällungsreaktion werden Verunreinigungen durch Fucoidan ausgeschlossen, so daß sich die Reinheit des gewonnenen Alginats im Vergleich mit herkömmlichen Alginaten verbessert.

Die unmittelbare Verarbeitung von Algenmaterial besitzt eine Reihe von Vorteilen. Erstens werden Nachteile bei der herkömmlichen Algenernte, die sich durch Zersetzung und Verrottung am Ernteort und die konservierende Chemikalienbehandlung ergeben, vollständig vermeidbar. Zweitens lassen sich die geernteten Algen nach Organen oder Gewebeabschnitten trennen, bevor die Alginatgewinnung durchgeführt wird. Da sich die verschiedenen Algengewebe durch verschiedene Verhältnisse der monomeren Mannuronsäure und Guluronsäure unterscheiden, können gezielt hochgereinigte Alginate mit einer bestimmten Mannuron-Guluron-Zusammensetzung hergestellt werden. Entsprechendes gilt für die Wahl bestimmter Gewebeteile zur Erzielung eines bestimmten Molekulargewichts. Es lassen sich erfindungsgemäße Alginatzusammensetzungen mit einem Molekulargewicht von mehr als 1.000 kD gewinnen. Schließlich kommt das erfindungsgemäße Verfahren ohne aufwendige Zentrifugationsschritte aus, wodurch die praktische Implementierung weiter vereinfacht wird. Die gezielte Gewinnung verschiedener Sorten hochreinen Alginats (z.B. guluronat- bzw. mannuronatreiches Alginat) läßt sich auch durch gezielte Gewinnung aus bestimmten Algenspezies *(Laminarales, Ectocarpales, Fucales* und anderen Alginat enhaltenden Algenspezies) realisieren, die sich durch die chemische Struktur des jeweiligen Alginats unterscheiden. Bei der organspezifischen Selektion werden hingegen Phylloide, Cauloide und Rhizoide der Algen getrennt und separat verarbeitet. Diese Differenzierung kann weiter verfeinert werden, in dem einzelne Gewebe der verschiedenen Organe getrennt und das Alginat aus diesen Geweben separat gewonnen wird.

Die Erfindung ist insbesondere zur Alginatgewinnung aus frischem oder getrockneten Algenmaterial (insbesondere Braunalgen) angepaßt. Es ist jedoch auch möglich, mit dem erfindungsgemäß Verfahren kommerziell verfügbare Rohalginate zu reinigen oder das erfindungsgemäß Verfahren mit bestimmten Wasch- oder Trennschritten (z.B. Zentrifugation) zu kombinieren, die aus den herkömmlichen Reinigungsverfahren bekannt sind.

Vorteile der Erfindung ergeben sich auch daraus, daß das hochreine Alginat unmittelbar aus den Algenpflanzen unter Kontrolle sämtlicher Prozeßschritte gewonnen wird. Es werden toxische Chemikalien (insbesondere Lösungsmittel) vermieden, so daß ein Einsatz für pharmazeutische Zwecke ohne weiteres möglich ist. Das Aufschäumen des ausgefällten Alginats stellt ein besonders einfaches und energiearmes Abtrennverfahren dar.

Gemäß einer bevorzugten Ausführungsform der Erfindung werden die folgenden Verfahrensschritte zunächst an Algenfrischmaterial oder getrocknetem Material durchgeführt und anschließend an hochgereinigtem Alginat teilweise wiederholt, das beim ersten oder früheren Verfahrensabläufen gewonnen wurde. Das Algenmaterial bzw. kommerzielle Alginat wird im folgenden als Ausgangsmaterial bezeichnet.

Erfindungsgemäß wird das Ausgangsmaterial zunächst in Gegenwart von Komplexbildnern, ggf. in einer Sodalösung (siehe unten, Beispiel 1), extrahiert. Anschließend werden in der Lösung vorhandene Zellbestandteile und Partikel durch Zugabe eines Granulats und falls erforderlich durch Zugabe von Ionenaustauschern (wie z.B. Amberlit) zur Sedimentation gebracht und die Lösung anschließend gefiltert. Die Sedimentation kann alternativ unter Verwendung von Elektrographit (z.B. in Kügelchen-Form) vorgenommen werden. Dabei wird Elektrographit in die Lösung eingerührt und durch einen Stromfluß zwischen zwei in die Lösung eingehängten Elektroden aufgeladen. Die geladenen Elektrographitkügelchen zeigen eine Akkumulation von Verunreinigungen, die hier die betreffenden Zellbestandteile und Fremdpartikel umfassen. Die Sedimentation wird vorzugsweise bei der Reinigung kommerzielen Alginatmaterials eingesetzt, kann aber anwendungsabhängig auch ganz wegfallen (siehe unten, Beispiel 2). Der Filterschritt kann eine mehrfache Filterung mit schrittweise sich verringernder Porengröße z.B. von 15 µm bis 0.1 µm umfassen. Aus der gefilterten Lösung wird das Alginat durch ein geeignetes Fällungsmittel ausgefällt. Die Fällung wird vorzugsweise mit einem Alkohol (z.B. Ethanol) durchgeführt. Es kann aber auch eine Säure oder ein anderes geeignetes Fällungsmittel verwendet werden. Die Alkoholkonzentration wird im Bereich zwischen 10% bis 50%, vorzugsweise im Bereich von 30% bis rd. 50% gewählt. In diesem Konzentrationsbereich bleiben Verunreinigungen durch immunologisch aktive Polysaccharide wie z.B. Fucoidan in der Lösung und können somit vom Alginat getrennt werden. Falls höhere Alkoholkonzentrationen wie z.B. beim Verfahren nach De Vos et al. verwendet werden, so können diese unerwünschten Polysaccharide nicht abgetrennt werden. Während der Ausfällung erfolgt vorzugsweise eine Durchströmung der Lösung mit einem Treibgas (z.B. Luft). Das ausgefällte Alginat wird durch die eingeblasene Luft nach oben aufgetrieben und kann von der Lösungsoberfläche leicht mit einer geeigneten Einrichtung (z.B. Netz, Sieb oder dergl.) von der Lösung abgetrennt werden. Anschließend wird das gesammelte Alginat mit einer Filterpresse entwässert.

Die genannten Verfahrensschritte werden anwendungsabhängig ganz, teilweise oder in teilweise modifizierter Form wiederholt. Nach dem letzten Verfahrensablauf wird das hochgereinigte Alginat in Ethanol und ggf. anschließend in Wasser gewaschen und bei Raumtemperatur luftgetrocknet.

Das gereinigte Alginat besitzt in Abhängigkeit vom Ausgangsmaterial ein Verhältnis der Monomeren Mannuronsäure und Guluronsäure im Bereich von 0.1 - 9 (entsprechend 1% bis 90% Mannuronsäure) und ein mittleres Molekulargewicht von ca. 10 kD bis mehr als 1000 kD. Derart gereinigtes, bei einer autoimmundiabetischen BB/OK Ratte implantiertes Alginat löst nach einer Implantationszeit von 3 Wochen keine oder nur eine sehr schwache Fremdkörperreaktion aus, wie im einzelnen unten erläutert wird.

Das beschriebene Verfahren zur Alginatgewinnung kann in Bezug auf das Fällungsmittel, die Wahl des Sedimentationsmittels, die Wahl des inerten Treibgases, das Fällungsverfahren und/oder das Vorgehen beim Einsammeln des ausgefällten Alginats modifiziert werden. Anstelle des zur Sedimentation eingesetzten Kieselgur kann auch jedes andere absorbierende Material wie Elektrographite, Granulate, Cellulose, poröse Recycling-Materialien in Pulver- oder Partikelform verwendet werden. Es ist auch möglich, zur Sedimentation ein mit einem absorbierenden Material beschichtetes Rührwerkzeug zu verwenden.

Gegenstand der Erfindung ist auch ein hochgereinigtes Alginat, das als Mischpolymer aus Mannuronsäure und Guluronsäure in einem Verhältnis im Bereich von 1% bis 90%, insbesondere rd. 70%, besteht und die in Anspruch 14 definierten Eigenschaften aufweist.

Erfindungsgemäß hergestelltes Alginate zeichnen sich aufgrund ihrer extremen Reinheit und aufgrund der schonenden Extraktion beim erfindungsgemäßen Verfahren durch charakteristische Stoffeigenschaften aus, die bei herkömmlichen Alginaten nicht gegeben sind. Diese Stoffeigenschaften umfassen sowohl charakteristische Parameter, die als Eigenschaften des Alginats direkt meßbar sind (z.B. Viskosität), als auch Parameter, die als Eigenschaften der erfindungsgemäß entfernten Verunreinigungen, deren vollständiges Fehlen oder vernachlässigbar geringes Auftreten auf die hohe Reinheit des erfindungsgemäßen Alginats hinweisen (z.B. Fluoreszenzeigenschaften von Verunreinigungen, Auslösung immunologischer Reaktionen bei Tierversuchen und in Zellkulturen etc.)

Ein erfindungsgemäß hergestellter Alginat zeichnet sich durch eine hohe Viskosität aus. Eine wässrige Lösung eines erfindungsgemäßen Alginats mit einer 0.1-%igen Konzentration besitzt eine Viskosität im Bereich von 10 bis 15 mPa s. Dies stellt einen erheblich höheren Wert gegenüber der Viskosität herkömmlicher Alginatlösungen gleicher Konzentration (rd. 1 bis 5 mPa s) dar. Bei 0.5%igen Lösungen erfindungsgemäß hergestellter Alginate ergibt sich eine Viskosität von 280 mPa s. Die Viskosität der Alginatlösungen werden mit einem Kugelrollviskosimeter (Typ: AMV-200, Anton Paar KG, Graz, Österreich) bestimmt. Aus den Viskositätswerten wird mittels der Verfahren nach Huggins ("J. Am. Chem. Soc.", Bd. 64, 1942, S. 2716 ff.) und Krämer ("Ind. Eng. Chem.", Bd. 30, 1938, S. 1200 ff.) das Molekulargewicht bestimmt. Es ergeben sich bei den erfindungsgemäß hergestellten Alginaten mittlere Molekulargewichte größer als 350 kD.

Erfindungsgemäß hergestellter Alginate sind frei von Phenolen und phenolähnlichen Verbindungen, insbesondere von Polyphenolen, die sich aus Phloroglucinol zusammensetzen, und Phenol-Protein-Verbindungen. Bei einer Anregungswellenlänge von 366 nm besitzen erfindungsgemäß hergestellte Alginate, abgesehen von einer Lösungsmittelfluoreszenz (Raman-Bande des Wassers) bei 418 nm im Spektralbereich von 380 bis 550 nm keine Fluoreszenzemissionen. Die Phenol- und Polyphenol-Freiheit erfindungsgemäß hergestellter Alginate ergibt sich auch aus Farbtests unter Verwendung der Folin-Denis-Reaganz oder mit Dimethoxybenzaldehyd (DMBA). Erfindungsgemäß hergestellte (Alginate besitzen bei diesen Farbtests, abgesehen von der Lösungsmittelabsorption, keine Extinktion.

Erfindungsgemäß hergestellte Alginate sind praktisch frei von Substanzen (z.B. Proteinen), die bei einer Wellenlänge von 350 nm absorbieren. Die Proteinfreiheit zeigt sich wiederum bei einer Fluoreszenzmessung mit einer Anregungswellenlänge von 270 nm, die, abgesehen von der Lösungsmittelfluoreszenz, im Spektralbereich von 300 bis 500 nm keine Fluoreszenzemission ergibt. Die Proteinfreiheit erfindungsgemäß hergestellter Alginate wird auch mit dem photometrischen Proteinnachweis nach Bradford ("Anal. Biochem.", Bd. 72, 1976, S. 248 ff.) gezeigt.

Erfindungsgemäß hergestellte Alginate sind auch Endotoxin-frei. Endotoxine sind Verunreinigungen, die bei einer Implantation einer Immunreaktionreaktion des Empfängers auslösen können, welche aus den Zellenwänden der bakteriellen Begleitflora der Alginate in herkömmliche Algenextrakte gelangen. Erfindungsgemäße Alginatlösungen (Konzentration: 0,25%) besitzen einen Endotoxingehalt von weniger als 14,5 Endotoxineinheiten pro Milliliter Alginatlösung (Meßverfahren: quantitave Endotoxinbestimmung mit Limulus Amöbozyten Lysat-Test).

Erfindungsgemäß hergestellte Alginate sind im Gegensatz zu herkömmlichen Alginatextrakten biokompatibel, soweit dies durch die unten erläuterten XTT- und MTT-Tests und Versuche an BB/OK-Ratten gezeigt wird.

Bevorzugte Anwendungen eines derartigen, hochgereinigten Alginats sind die Transplantationschirurgie, bei der lebende Zellen in einer Alginatkapsel eingeschlossen und ohne die Auslösung immunologischer Reaktionen im Körper eines Lebewesens implantiert werden. Das hochgereinigte Alginat kann auch in den Gebieten der Lebensmittel- oder Textiltechnik zur Erhöhung der Verträglichkeit bestimmter Nahrungsmittel oder Stoffe eingesetzt werden. Es wird betont, daß das oben erläuterte erfindungsgemäße Verfahren auch zur Herstellung hochgereinigten Alginats mit geringerem Molekulargewicht bis zu 1000 kD geeignet ist.

Weitere Vorteile und Einzelheiten der Erfindung werden im folgenden, insbesondere unter Bezug auf die beigefügten Figuren beschrieben. Es zeigen:
- Fig. 1: eine Schnittansicht eines Düsenkopfes zur Herstellung von Alginatkapseln,
- Fig. 2: Fluoreszenzspektren zur Demonstration der Phenolfreiheit erfindungsgemäß hergestellter Alginate,
- Fig. 3: Fluoreszenzspektren zur Demonstration der Proteinfreiheit erfindungsgemäß hergestellter Alginate, und
- Fig. 4: Ergebnisse eines Lymphozytenstimulationstests zur Demonstration der Immunogenfreiheit erfindungsgemäß hergestellter Alginate.

### Ausführungsbeispiele

Im folgenden werden konkrete Ausgestaltungen der oben allgemein erläuterten Verfahrensweise an Beispielen beschrieben. Dabei wird ohne Beschränkung auf die Reinigung bzw. Verwendung von Alginatmaterial auf der Basis von Braunalgen Bezug genommen. Anstelle von Braunalgen können allgemein alle alginatenthaltenden Salzwasser- oder Süßwasser-Algen verwendet werden. Die Reinigung von Alginatmaterial aus anderen Algen erfolgt in entsprechender Weise.

Das Ausgangsmaterial umfaßt (1) frische Braunalgen, (2) getrocknete Braunalgen oder (3) kommerzielles Alginat. Als Frischmaterial (1) werden in der Natur oder in einem Kultivierungsraum oder Gewächshaus geerntete Braunalgen oder Braunalgenteile aus vorbestimmten Entwicklungsstadien des Lebenszyklus der Algen oder entsprechendes in einem homogenen Bioreaktor kultiviertes Algenmaterial verwendet. Das Trockenmaterial (2) besteht aus getrockneten Braunalgen, die entsprechend diesen Alternativen gewonnen wurden.

### Beispiel 1

Beim ersten Beispiel wird auf 80 g trockene und anschließend wieder hydratisierte (gewässerte) Algen Bezug genommen. Bei kommerziellem Alginat, dessen Trockengewicht nur rund 1% des Frischgewichts ausmacht, wird entsprechend weniger Trockengewicht eingesetzt. Das trockene Material (z.B. *Laminarales, Fucales)* gemäß (2) oder (3) wird je nach Ausgangsmenge für mehrere Stunden in warmem Leitungswasser gewässert. Dies kann beispielsweise bei Leitungswasser mit einer Temperatur von 40°C mindestens 3 bis 4 Stunden dauern, wobei das Wasser mehrfach ausgetauscht wird oder fließt. Bei Verwendung von kälterem Wasser muß die Wässerung entsprechend verlängert werden. Die Wässerung erfolgt vorzugsweise dadurch, daß das Material in einem wasserdurchlässigen Behältnis (z.B. wasserdurchlässiger Sack) in fließendes Leitungswasser gehängt wird. Bei Wässerung in stehendem Wasser wird das Material z. B. bei einem Ausgangstrockengewicht von rund 80 g getrockneter Algen (entsprechend 10% des Frischgewichts von rund 800 g) in 3 bis 6 1 Wasser gewässert. Nach der Wässerung erfolgt die Verfahrensweise für alle drei genannten Arten von Ausgangsmaterial (1) bis (3) analog.

Das Material wird in rund 5.7 l einer 25 mM EDTA-Lösung (Aqua dest. bzw. demineralisiertes Wasser) suspendiert (bzw. im Falle von kommerziellem Alginat (3) gelöst). Die Einwirkung der Ethylendiamintetraessigsäure(EDTA)-Lösung erfolgt mindestens 10 Stunden. Die Einwirkungszeit kann verkürzt werden, wenn die Suspension laufend gerührt wird. Mit steigender Einwirkungszeit verbessert sich die Ausbeute an gereinigtem Alginat. Bei einer ungerührten Suspension kann beispielsweise eine Einwirkungszeit von mehreren Tagen vorgesehen sein.

Anschließend werden 5% Na₂CO₃ und EDTA als Festsubstanzen unter Rühren zugegeben. Die EDTA-Menge wird derart gewählt, daß eine 50 mM-EDTA-Lösung gebildet wird. Die Suspension wird solange gerührt, bis eine homogene (feindisperse) Lösung vorliegt. Dieser Zustand ist insbesondere dann erreicht, wenn in der Lösung nur noch wenige Zellbestandteile sichtbar sind. Anschließend werden rund 34 g Kieselgur unter Rühren zugesetzt und die Lösung für mindestens 2 Tage gerührt. Es ist alternativ möglich, das Kieselgur zusammen mit Na₂CO₃ und EDTA unter Rühren zuzugeben. Es kann anwendungsabhängig (insbesondere in Abhängigkeit vom verwendeten Braunalgenmaterial) vorgesehen sein, zusätzlich Ionenaustauschermaterial gleichzeitig mit dem Kieselgur oder Elektrographit zuzugeben. Es ist beispielsweise möglich, zusätzlich 34.2 g Amberlit als Ionenaustauscher zuzusetzen, das vorher einer Reinigung unterzogen worden ist. Diese Reinigung dient der Entfernung toxischer Substanzen und umfaßt eine Wässerung in fließendem Wasser (Dauer rund 3 Stunden).

Nach der Kieselgurbehandlung wird das Volumen der Lösung mit demineralisiertem Wasser auf 22.8 l verdünnt, um die Viskosität zu verringern. Die Verdünnung wird allgemein derart gewählt, daß die Lösung anschließend filtrierbar ist. Der Wasserzusatz hängt somit insbesondere auch vom verwendeten Braunalgenmaterial ab. Nach der Verdünnung und kurzzeitigem Durchrühren wird die Lösung für mindestens 10 Stunden stehengelassen, um feste Bestandteile sedimentieren zu lassen. Die Standzeit kann auch im Bereich von Tagen liegen. Der Überstand wird abdekantiert und filtriert. Die Filtration erfolgt mehrstufig, wobei erst ein Tiefenfilter mit einer Porengröße von 15 µm und anschließend ein Filter mit einer Porengröße von 0.1 µm verwendet wird.

Anschließend folgt ein Salzzusatz zu dem Filtrat. Es wird der Zusatz von KCl bevorzugt, wobei anwendungsabhängig auch andere, entsprechende Salze eingesetzt werden können. Es wird soviel KCl als Festsubstanz zugegeben, daß sich eine 0.13 M KCl-Lösung ergibt.

Anschließend folgt eine Ethanol-Fällung. Die Menge des Ethanolzusatzes hängt davon ab, wieviel Fucoidan sich im Material befindet. Bei der ersten Ethanolfällung sollte die Ethanolendkonzentration bei Anwesenheit von Fucoidan nicht 40% überschreiten, da sonst das Fucoidan mit ausfällt. Beim angegebenen Beispiel werden rund 12 1 99%iger Ethanol zugesetzt, so daß die Endkonzentration des Ethanol in der Gesamtlösung bei rund 34% liegt. Es kann auch vorgesehen sein, daß die Ethanolkonzentration in Abhängigkeit von der Art des Ausfällens des Alginats gewählt wird. Durch Variation der Ethanolkonzentration kann erzielt werden, daß das Alginat fadenförmig oder watteförmig ausfällt. Eine derartige Ausfällung wird nach Möglichkeit angestrebt, damit das Alginat aufschwimmt bzw. weiterverarbeitet werden kann, wie dies unten erläutert wird.

Anstelle von Ethanol kann auch mindestens ein anderer Alkohol (z. B. Isopropanol) oder eine Fällungssäure zugegeben werden, wobei die Konzentration sich nach den genannten Kriterien richtet.

Während der Fällung erfolgt eine Durchströmung der Lösung mit einem Treibgas (z.B. Luft). Das ausgefällte Alginat wird während der Fällung, d.h. im Entstehen, durch die eingeblasene Luft nach oben aufgetrieben und kann von der Lösungsoberfläche leicht mit einer geeigneten Einrichtung (z.B. Netz, Sieb, oder dgl.) von der Lösung abgehoben werden. Das ausgefällte Alginat kann auch ohne Treibgaszusatz durch Dekantieren oder Umrühren mit einer Rühreinrichtung, an der ausgefälltes Alginat haften bleibt, gesammelt werden. Anschließend wird das gesammelte Alginat mit einer Filterpresse entwässert, um dem stark hygroskopischen Material zumindest teilweise Wasser zu entziehen.

Die bis hier realisierten Verfahrensschritte werden anwendungsabhängig ganz, teilweise oder teilweise unter modifizierten Bedingungen wiederholt. Danach wird eine Weiterverarbeitung des gefällten Alginats wie folgt durchgeführt.

Das gefällte Alginat wird in 11.4 l und 0.5 M-KCl/10 mM-EDTA-Lösung aufgelöst. Die Auflösung erfolgt unter Rühren, bis eine homogene Lösung vorliegt. Anschließend folgt eine zweite Fällung mit rund 10 1 einer 99%igen Ethanollösung unter Treibgaszuführung. Unter diesen Bedingungen beträgt die Alkoholkonzentration in der Gesamtlösung rund 44%. Bei dieser zweiten Fällung kann eine höhere Alkohol-(bzw. Säure-)Konzentration gewählt werden, da das Fucoidan (siehe oben) bereits abgetrennt ist. Allerdings wird die Konzentration des Fällungsmittels wiederum so eingestellt, daß die Bildung von faden- oder watteartigem Alginat gefördert wird. Bei nichtoptimaler Alkoholkonzentration ist das Alginat gelatineartig und kann somit nicht flotiert werden (Aufschwimmen unter Treibgaswirkung).

Anschließend wird das gefällte Alginat wieder durch eine Filterpresse entwässert und mehrmals mit der 10-fachen Menge einer 70%igen Ethanollösung gewaschen. Anschließend wird das Material bei Raumtemperatur getrocknet. Die Trocknung kann anwendungsabhängig unter sterilen Bedingungen erfolgen. Es kann nach der Waschung mit Ethanol vorgesehen sein, das Material mehrfach mit demineralisiertem Wasser zu waschen oder gegen demineralisiertes Wasser zu dialysieren, um Restspuren von Begleitstoffen zu entfernen.

Die Zahl der erfindungsgemäß durchgeführten Fällungen richtet sich nach den Verunreinigungen bzw. den toxischen Beiprodukten im Ausgangsmaterial.

Die Gewebeverträglichkeit des entsprechend dem Beispiel gewonnenen Alginats wird wie folgt geprüft. Es werden Implantationsexperimente mit normoglykämischen (6.1 + 0.4 mM Plasmaglucose), diabetisanfälligen BB/OK-Ratten (200 + 25 Tage alt) durchgeführt. Diese Ratten besitzen eine erheblich höhere Empfindlichkeit gegen Verunreinigungen in Alginaten als die oben genannten Lewisratten.

Ba²⁺-Alginatkapseln wurden aus dem hochgereinigten Alginat entsprechend der Verfahrensweise hergestellt, die in DE-OS 42 04 012 Al beschrieben ist. Die Alginatkapseln besitzen einen mittleren Durchmesser von 200 µm bis 400 µm. Die Implantation erfolgte unter die Nierenkapsel der BB/OK-Ratten. Die Empfängertiere blieben normoglykämisch und zeigten keinen Verlust an Körpergewicht. Nach drei Wochen wurden die Tiere getötet, die Nieren herausgenommen und in Bouin'scher Lösung fixiert. Nach Einbettung in Paraffin folgte die Präparation von 7 µm-Schnitten. Jeder 20. Schnitt von zwei unabhängigen Individuen wurde zur histologischen Untersuchung herangezogen.

Das Ergebnis der Untersuchung für verschiedene Proben im Vergleich mit Rohalginat ist in der folgenden Tabelle zusammengestellt:
Resultat für zwei unabhängige Proben:

| Probe | Ratte # | Histologische Beurteilung (Fibrose) | Endotoxingehalt einer 0,25%igen Lösung des Alginats |
|---|---|---|---|
| S1 | V159 | (+) | 1 EU/ml |
| | V161 | + | |
| S2 | V454 | 0 | 3,5 EU/ml |
| Rohalginat | V12 | ++++ | > 1000 EU/ml |
| (Legende: 0: keine Reaktion, (+): sehr schwache Reaktion, +: schwache Reaktion, ++++: sehr starke Fibrose) | | | |

Es zeigt sich, daß die Implantation mit erfindungsgemäß hergestelltem Alginat keine oder nur eine sehr schwache Reaktion auslöst, wohingegen bei Implantation mit kommerziell angebotenem Rohalginat eine sehr starke Fibrose auftritt.

Der Endotoxingehalt, der charakteristisch für einen potentiellen Bakterienbefall ist, zeigt im Falle der hochgereinigten Alginate hervorragende, nahezu vernachlässigbare Werte, wohingegen der Vergleichswert des kommerziellen Rohalginats rd. tausendfach größer ist.

Am erfindungsgemäß hergestellten, hochgereinigten Alginat wurde auch unter Anwendung der folgenden Testverfahren festgestellt, daß keine Verunreinigungen von toxischen Substanzen gegeben ist. Die Testverfahren umfassen insbesondere fluoreszenzspektroskopische Verfahren und Endotoxin- oder Mitogenaktivitäts-Essays, wie sie von G. Klöck et al. in "Appl. Microbiol. Biotechnology" (Band 40, 1994, Seite 638 ff) und in "Biomaterials" (Band 18, 1997, Seite 707) beschrieben sind, NMR-spektroskopische Verfahren, die Bestimmung des antioxidativen Potentials des hochgereinigten Alginats durch Ermittlung der Reaktion auf Zugabe von HOCl und über die Bestimmung der oxidativen Aktivität neutrophiler Granolycyten unter Zuhilfenahme der Chemolumineszenz (siehe K. Arnold in "Abhandlungen der sächsischen Akademie der Wissenschaften zu Leipzig", Band 58, 1997, Heft 5) und das Verfahren der "Free Flow Electrophoresis" wie es von U. Zimmermann et al. in "Electrophoresis" (Band 13, 1992, Seite 269) beschrieben ist. Eine toxische Verunreinigung wurde mit diesen Verfahren nicht bestimmt.

Nach den in der oben genannten Publikation von G. Klöck et al. (1997) angegebenen Verfahren wurde ferner das Verhältnis von Mannuron- zu Guluronsäure mit Hilfe der sogenannten "Circular Dichroismus-Spectroscopy" bzw. mit der IR-Spektroskopie ermittelt. Ferner wurde auch das Molekulargewicht über die Bestimmung der Viskosität ermittelt.

Die mit Ba²⁺ vernetzten Kapseln aus Alginat besitzen eine hervorragende Elastizität, wie es mit Hilfe von Kompressionsmessungen nachgewiesen werden konnte.

### Beispiel 2

Beim zweiten Beispiel wird auf 10 g trockene Algen Bezug genommen. Bei größeren Ausgangsmassen sind die im folgenden gegebenen quantitativen Größen entsprechend linear umzurechnen. Im Unterschied zu Beispiel 1 erfolgt die Alginatgewinnung bzw. -reinigung vorteilhafterweise ohne eine Wässerung oder Quellung. Das trockene Ausgangsmaterial wird vielmehr unmittelbar in eine EDTA-Lösung gegeben. Die EDTA-Lösung besitzt eine Konzentration im Bereich von rd. 10 bis 50 mM. Die Suspension wird 24 Stunden gerührt und anschließend zur Entfernung von Festmaterial gesiebt. Ein weiterer Vorteil der beim zweiten Beispiel erläuterten Verfahrensweise besteht darin, daß der EDTA-Verbrauch und auch der Alkoholeinsatz verringert wird.

Es kann vorgesehen sein, daß der Suspension während der EDTA-Behandlung zusätzlich Aktivkohle zugesetzt wird. Die Masse der zugesetzten Aktivkohle beträgt vorzugsweise etwa 10 bis 200 % der eingewogenen Algentrockenmasse.

Anschließend erfolgt unmittelbar ohne einen gesonderten Sedimentationsschritt eine Filtration der Suspension. Die Filtration erfolgt zweistufig, wobei erst ein Vor- oder Tiefenfilter mit einer Porengröße von 15 µm und anschließend ein Filter mit einer Porengröße von 0.2 µm verwendet wird.

Nach einem Salzzusatz zu dem Filtrat wie bei Beispiel 1 (z. B. Bildung einer 0.13 M KCl-Lösung) folgen mehrere Ethanol-Fällungen. Für die erste Fällung wird 99%iger Ethanol (vergällt mit Aceton) zugesetzt, so daß sich eine Endkonzentration des Ethanol in der Gesamtlösung von 37.5% ergibt. Das Fällungsprodukt wird aufgesammelt und in eine 0.5M KCl-Lösung (ohne EDTA) gegeben. Das Volumen der KCl-Lösung wird auf ein Drittel des Lösungsvolumens vor der Fällung eingestellt.

Für die zweite Fällung wird wiederum 99%iger Ethanol (vergällt mit Aceton) zugesetzt, so daß sich hier eine Endkonzentration des Ethanol in der Gesamtlösung von 44% ergibt. Das Fällungsprodukt wird aufgesammelt und in bidestilliertes Wasser mit einem Volumen entsprechend dem Volumen der KCl-Lösung nach der ersten Fällung gegeben.

Vor der dritten Fällung kann eine Dialyse des bei der zweiten Fällung gewonnenen Fällungsprodukts durchgeführt werden. Die Dialyse, die kein zwingendes Verfahrensmerkmal ist, erfolgt für die Dauer von 3 Tagen mit 3 Wasserwechseln pro Tag. Für die dritte Fällung wird wiederum 99%iger Ethanol (vergällt mit Aceton) zur Einstellung einer Endkonzentration des Ethanol in der Gesamtlösung von 50% zugesetzt. Das Fällungsprodukt wird aufgesammelt und getrocknet.

### Beispiel 3

Im folgenden wird ein Beispiel zur Transplantationschirurgie, nämlich die Mikrokapsulierung Langerhans'scher Inseln, beschrieben.

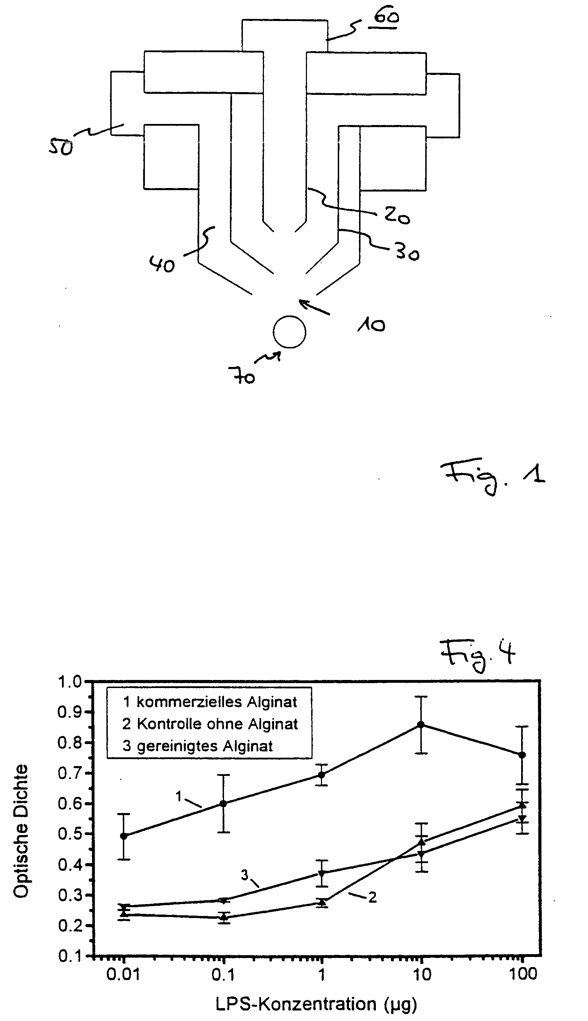

Isolierte Langerhans'sche Inseln wurden in einer Lösung von 0.9% NaCl und 0.5% des gereinigten Alginats suspendiert. Diese Suspension wurde durch eine in Fig. 1 gezeigte Sprühdüse 10 fein zertropft. Die Düse hat ein bewegliches inneres Hohlrohr 20 (innere Düse) mit Lüranschluß (innerer Durchmesser 350 µm bzw. 2 mm), ein mittleres Hohlrohr 30 (mittlere Düse) mit einem Durchmesser von 1 mm bzw. 3.5 mm und einen äußeren Kanal 40, der in einem justierbaren Luftfokussierkopf 50 mündet. Diese Elemente wurden an einem Düsenkopf 60 montiert, an dem sich Druckluft- und Lüranschluß befinden.

Die Inselsuspension in Alginat wird durch den zentralen Düsenkanal gedrückt. Durch den umgebenden Kanal wird eine Lösung von 0.5 bis 2% des Alginats in 0.9% Kochsalzlösung appliziert. Bei den am Düsenausgang entstehenden Tropfen umschließt auf diese Weise die äußere Alginatlösung (0.5 bis 2%) die in der 0.5%igen Alginatlösung suspendierten Inseln. Durch den äußeren, dritten Kanal wird Druckluft zugeführt, welche die Tropfen von der Düsenöffnung abschert. Die Druckluft wurde auf 30 bis 40 mbar (7 bis 8 1/min) eingestellt. Die Alginattropfen 70 wurden in 40 mL Vernetzerlösung mit 20 mM BaCl₂ und 5 mM Histidin geliert. Die Vernetzerlösung ist mit NaCl auf eine physiologische Konzentration (290 mOsmol) eingestellt. Die Kapseln wurden dann dreimal entsprechend mit isotoner Kochsalzlösung gewaschen.

Mit dem erfindungsgemäß hergestellten Alginat können allgemein Umhüllungen für allogene und xenogene Gewebe, insbesondere endokrines Gewebe, hergestellt werden.

### Weitere Charakterisierung erfindungsgemäß hergestellter Alginatmaterials

### 1. Fluorimetrischer Phenolnachweis

Erfindungsgemäß hergestellte Alginate enthalten keine störenden Verunreinigungen auf der Basis von Phenolen, Polyphenolen und anderen Phenolverbindungen. Diese Phenolfreiheit bedeutet, daß die genannten Verunreinigungen nicht oder in einem derart geringen Gehalt in den Alginaten enthalten sind, daß Anwendungen in der Biologie und Medizin, insbesondere die obengenannten Anwendungen, nicht durch Immunreaktionen oder dergleichen gestört werden. Die Phenolfreiheit wird mit einer fluorimetrischen Analyse nachgewiesen, die von G. Skajk-Braek et al. (s. "Biotechnology and Bioengineering", Bd. 33, 1989, S. 90 ff.) beschrieben worden ist. Die Fluoreszenzmessung wird mit einem Spektrometer LS50 (Perkin-Elmer, Beaconsfield) durchgeführt. Bei einer Anregungswellenlänge von 366 nm ergeben sich die in Fig. 2 gezeigten Fluoreszenzspektren an Lösungsproben während der Reinigung gemäß Beispiel 2. Vor und nach der anfänglichen Filtration zeigt die Alginatlösung eine starke Fluoreszenz im Bereich zwischen 380 und 550 nm (obere Spektren). Nach den Filtrations- und Fällungsschritten ist die Fluoreszenz erheblich vermindert. Die Konzentration der Endlösung beträgt rd. 0,2-0,3%. Es verbleibt lediglich ein Fluoreszenzmaximum bei 418 nm, was der Lösungsmittelfluoreszenz entspricht. Im erfindungsgemäß gereinigten Alginat ist die Fluoreszenz der Phenole und Phenolverbindungen auf weniger als rd. 10% gegenüber der ungereinigten Alginatlösung reduziert.

Fig. 2 zeigt, daß die Phenolgehalte im Laufe des erfindungsgemäßen Verfahrens drastisch abnehmen und daß das gereinigte Alginat eine Emission zeigt, die nur noch geringfügig über den Werten hochreinen Wassers liegt.

### 2. Fluorimetrischer Proteinnachweis

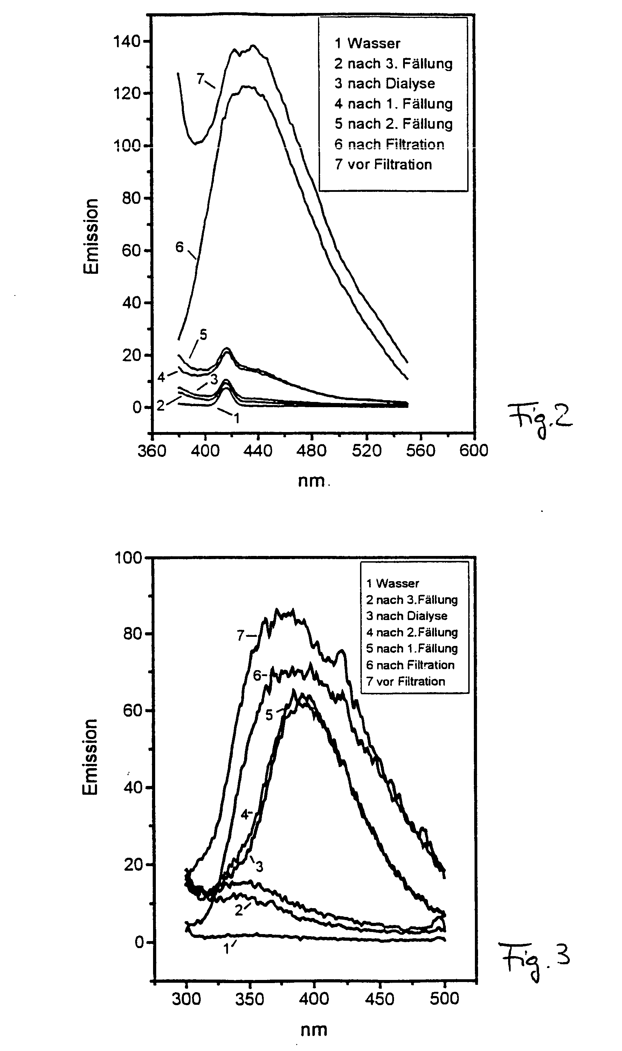

Die Proteinfreiheit erfindungsgemäß hergestellter Alginate wird wie bei der Analyse gemäß 1. fluorimetrisch nachgewiesen. Fig. 3 zeigt, daß bei einer Anregungswellenlänge von 270 nm vor der Reinigung eine starke Emission im Bereich von 300 bis 500 nm gemesen wird. Diese Emission fällt im Laufe der Reinigung auf einen Wert unterhalb von 20% der Fluoreszenz der ungereinigten Alginatlösung ab. Nach der letzten Fällung ist die Fluoreszenz nicht mehr nachweisbar oder vernachlässigbar klein.

### 3. Phenolnachweis nach Folin-Denis bzw. mit DMBA

Der Folien-Denis-Nachweis färbt sämtliche phenolhaltige Verbindungen. Erfindungsgemäß hergestellte Alginate zeigen bei diesem Nachweise keine Extinktion. Der Folin-Denis-Nachweis wird unter den folgenden Bedingungen durchgeführt:

Die zu analysierende Alginatlösung (0,5 ml) wird mit 1 ml Methanol zur Extraktion versetzt. Die Probe wid nach intensivem Schütteln für ca. 8h in den Kühlschrank gestellt. Das ausgefallene Alginat wird abzentrifugiert, 10 µl des Überstandes werden in 40 µl des Folin-Denis Reagenz (Fluka, Deisenhofen, Deutschland), 80 µl 1 M Na₂CO₃ und 120 µl H₂O (Reihenfolge einhalten) gegeben. Nach intensivem Schütteln erfolgt die Farbentwicklung im Wasserbad bei 50°C für 30 min. Es folgt die Messung der optischen Dichte mit Hilfe eines Photometers bei einer Wellenlänge von 650 nm (oder 725 nm).

Auch beim Zusatz von Dimethoxybenzaldehyd (DMBA) zeigen die erfindungsgemäß hergestellter Alginate keine photometrisch auswertbare Farbreaktion. Dieser Test wurde unter den folgenden Bedingungen ausgeführt: Die zu analysierende Alginatlösung (0,5 ml) wird mit 1 ml Methanol zur Extraktion versetzt. Die Probe wird nach intensivem Schütteln für ca. 8h in den Kühlschrank gestellt. Das ausgefallene Alginat wird abzentrifugiert. Anschließend erfolgt die Zugabe von 10 µl des Überstandes in die DMBA Lösung, die wie folgt hergestellt wurde: 2 g DMBA (Fluka, Deisenhofen, Deutschland) werden in 100 ml Eisessig gelöst. 16 ml HCl (37%) werden mit Eisessig auf 100 ml aufgefüllt. Beide Lösungen sind kurz vor der Verwendung 1:1 zum Arbeitsreagenz zu mischen.

Es folgt die Messung der optischen Dichte mit Hilfe eines Photometers bei einer Wellenlänge von 490 nm (oder 510 nm) gegen eine Eichreihe Phloroglucinol (Sigma, Deisenhofen, Deutschland).

### 4. Proteinnachweis nach Bradford

Der Proteinnachweis nach Bradford (s. "Anal. Biochem.", Bd. 72, 1976, S. 248 ff.) basiert auf der Tatsache, daß der Farbstoff Coomassie brilliant blue sehr spezifisch an Proteine anbindet, wodurch es zu einem Farbumschlag von rot nach blau kommt. Die Intensität der Blaufärbung korreliert linear mit der Proteinmenge und kann photometrisch quantifiziert werden. Mit dieser Methode sind Proteine bis in den unteren µg-Bereich nachweisbar. Es wurden 10 µl einer 0,5%igen Alginatlösung untersucht. Als farbstoffhaltige Nachweissubstanz wurde das sogenannte Bradford-Reaganz (Hersteller Sigma, Steinheim, Deutschland) der Alginatlösung zugesetzt. Nach einer Inkubationszeit von 10 Minuten wurde die Probenabsorption photometrisch bei 570 nm unter Verwendung eines "Thermomex Microplate Reader"-Gerätes (Hersteller Molecular Devices, Manlow Park, USA) gemessen. In der erfindungsgemäßen Alginatlösung wurden mit diesen Verfahren keine Proteine gefunden.

### 5. XTT- und MTT-Tests

Die Biokompatibilität der erfindungsgemäß gereinigten Alginate kann mit den folgenden Tests gezeigt werden. Lebende Zellen, wie Maus-Lymphozyten oder Fibroblasten werden für einige Tage mit den Alginaten in Kultur gehalten. Weisen die Alginate eine immunogene Wirkung auf, so werden die Zellen stimuliert oder inhibiert (um den Effekt der Stimulation zu verdeutlichen können zusätzliche Stimulanzien, z.B. LPS oder PHA in die Kultur gegeben werden). Die bei Lymphozyten erhöhte und bei Fibroplasten verminderte Stoffwechselaktivität kann anhand der Umsetzung eines Farbstoffs (XTT oder MTT) durch mitochondrielle Dehydrogenasen visualisiert und photometrisch quantifiziert werden. Bei erfindungsgemäß gereinigten Alginaten ist die Farbstoffumsetzung vernachlässigbar gering (s. Fig. 4), wohingegen bei kommerziellen Alginaten eine starke Farbstoffumsetzung nachweisbar ist. Fig. 4 zeigt die Ergebnisse der Farbtests für verschiedene LPS-Vorstimulierungen.

Die Kultivierung der Zellen wurde unter den folgenden Bedingungen durchgeführt. Die Lymphozytenzellsuspension (1*10⁶ Zellen/ml in Complete Growth Medium) wird mit verschiedenen Konzentrationen an Lipopolysacchariden (LPS, Endkonzentration: 0,01 µg/ml) und Alginatlösung (Endkonzenration: 0,05%) versetzt und 3 Tage bei 5% CO₂ und 37°C kultiviert. Danach wird die Suspension mit einer Lösung des Tetrazoliumsalzes (XTT oder MTT) und für weitere 6h inkubiert. Es folgt die Messung der optischen Dichte bei 450 nm (Referenzmessung bei 650 nm).

### 6. Versuche in BB/OK-Ratten

Wie oben bei Beispiel 1 beschrieben, kann die Biokompatbilität erfindungsgemäß hergestellter Alginate auch durch in vivo-Tests an BB/OK-Ratten gezeigt werden, deren Immunsystem eine erhöhte Makrophagenaktivität aufweist. Erfindungsgemäß gereinigtes Alginat, das entsprechend den oben beschriebenen Schritten kapselförmig in Ratten-Nieren implantiert worden ist, verursacht nach 3 Wochen Inkubationszeit keine Fibrosen.

### 7. Elektrorotation

Die Biokompatbilität der erfindungsgemäß gereinigten Alginate kann auch mit folgendem Test gezeigt werden. Lebende Zellen, wie Human-Lymphozyten oder Maus-Lymphozyten werden für einige Tage mit Alginaten in Kultur gehalten. Weisen die Alginate eine immunogene Wirkung auf, reagieren die Zellen mit einer stark vergrößerten Membranoberfläche (Zunahme der Mikrovilli) (um diesen Effekt zu verdeutlichen können zusätzliche Stimulanzien, z.B. LPS oder PHA in die Kultur gegeben werden). Eine solche Zunahme läßt sich über eine Erhöhung in der spezifischen Membrankapzität Cₘ ermitteln. Die Elektrorotation der Zellen unter Wirkung hochfrequenter elektrischer Felder bietet eine Möglichkeit, diese Veränderungen der Lymphozytenmembran auf dem Einzelzellniveau nachzuweisen. Bei erfindungsgemäß gereinigten Alginaten läßt sich keine Erhöhung der spezifischen Membrankapazität messen, wohingegen bei kommerziellen Alginaten eine Erhöhung dieses Wertes nachweisbar ist.

Die Messungen wurden unter folgenden Bedingungen ausgeführt. Die Lymphozytenzellsuspension (1*10⁶ Zellen/ml in Complete Growth Medium) wird mit einer Alginatlösung (Endkonzentration: 0,05%) versetzt nd 3 Tage bei 5% CO₂ und 37°C kultiviert. Danach wird die Suspension 2 x in isoosmolaler Inositollösung (290 mOsm/kg) gewaschen, anschließend gut resuspendiert. Bei verschiedenen Leitfähigkeiten (10, 25, 40 µS/cm), eingestellt mit HEPES-KOH, pH 7,2) erfolgt die Bestimmung der charakteristischen Frequenz (Maximum) der Antifeldrotation mittels der Kompensationsmethode. Mit Hilfe einer linearen Regression der mit dem Zellradius normierten charakteristischen Frequenzen, aufgetragen gegen die externe Leitfähigkeit, kann man die elektrischen Parameter (Kapazität und Leitfähigkeit) der Plasmamembran bestimmen. Die elektrischen Parameter der in Anwesenheit erfindungsgemäß hergestellter Alginate kultivierten Zellen bleiben unverändert, wohingegen die mit kommerziellen Alginaten kultivierten Zellen eine Zunahme der Membrankapazität um 10 bis 50 % aufweisen.

### 8. Zellgröße und Zellzahl

Die Biokompatibilität der erfindungsgemäß gereinigten Alginate kann auch mit folgendem Test gezeigt werden. Lebende Zellen, wie Human-Lymphozyten oder Maus-Lymphozyten werden für einige Tage mit Alginaten in Kultur gehalten. Weisen die Alginate eine immunogene Wirkung auf, so vergrößern sich die Zellen und es erhöht sich die Anzahl der Zellen (um diesen Effekt zu verdeutlichen, können zusätzliche Stimulanzien, z.B. LPS oder PHA in die Kultur gegeben werden). Die Zellgröße und die Zellzahl kann mit Hilfe des CASY1 Cell Analyzer (Model TTC, Schärfe Technology, Reutlingen, Deutschland), der nach dem Coulter Prinzip arbeitet, ermittelt werden. Ein solches Gerät erlaubt die schnelle, genaue Erfassung (Größe, Größenverteilung und Zellzahl) mehrerer tausend Zellen pro Messung. Bei erfindungsgemäß gereinigten Alginaten läßt sich keine signifikante Zellvergrößerung und Erhöhung der Zellzahl nach Inkubation messen, wohingegen bei kommerziellen Alginaten eine deutliche Zellvergrößerung (rd. 50 %) und eine erhöhte Zellzahl (35 bis 50 %) nachweisbar ist.

Die Messungen wurden unter folgenden Bedingungen durchgeführt. Die Lymphozytensuspension (1*10⁶ Zellen/ml in Complete Growth Medium) wird mit einer Alginatlösung (Endkonzentration: 0,05%) versetzt und 3 Tage bei 5% CO₂ und 37°C kultiviert. Danach wird ein abzentrifugiertes und in Inosit (oder PBS) aufgenommenes Aliquot (100 µl) der gut resuspendierten Lymphozytensuspension 2 x in isoosmolaler Inositollösung (290 mOsm/kg) gewaschen und in 10 ml PBS (phosphatgepufferte Saline) aufgenommen und sofot im CASY1 gemessen. Aus den CASY-Histogrammen kann der Anteil der großen, stimulierten Zellen an der Gesamtpopulation berechnet werden.

## Patentansprüche

1. Verfahren zur Gewinnung einer hochgereinigten Alginatzusamensetzung aus einem Mischpolymer aus Mannuronsäure und Guluronsäure, wobei das mittlere Molekulargewicht des Mischpolymers größer als 350 kD ist, mit den Schritten:
a) Extrahieren von Algenmaterial in einer Lösung mit einem Komplexbildner,
b) Sedimentieren von Zellbestandteilen und Partikeln mit einem porösen Bindemittel,
c) Filtern der Lösung,
d) Ausfällen von Alginat aus der Lösung und
e) Sammeln und Entwässern des gefällten Alginats, wonach die Schritte a) bis e) ggf. mindestens einmal wiederholt werden und
f) Auflösen des Alginats und mindestens ein weiteres Ausfällen des Alginats.

2. Verfahren gemäß Anspruch 1, bei dem zum Extrahieren als Komplexbildner Ethylendiamentetraessigsäure verwendet wird.

3. Verfahren gemäß Anspruch 1, bei dem das Extrahieren in einer Sodalösung erfolgt.

4. Verfahren gemäß Anspruch 2 oder 3, bei dem zum Extrahieren der Lösung Aktivkohle zugesetzt wird.

5. Verfahren gemäß Anspruch 1, bei dem das Sedimentieren mit einem porösen Granulat auf der Basis von Kieselgur, Zellulose oder Recycling-Materialien aus nachwachsenden Rohstoffen erfolgt.

6. Verfahren gemäß einem der vorhergehenden Ansprüche, bei dem das Filtern mit Tiefenfiltern jeweils abnehmender Porengröße erfolgt.

7. Verfahren gemäß einem der vorhergehenden Ansprüche, bei dem das Ausfällen von Alginat mit Ethanol erfolgt.

8. Verfahren gemäß Anspruch 7, bei dem der Ethanolgehalt im Bereich von 10-50 % gewählt ist.

9. Verfahren gemäß einem der vorhergehenden Ansprüche, bei dem das Sammeln des ausgefällten Alginats durch Aufschäumen aus der Lösung, durch Dekantieren der Lösung oder durch Rühren der Lösung mit einer Rühr- und Sammeleinrichtung erfolgt.

10. Verfahren gemäß einem der vorhergehenden Ansprüche, bei dem das Entwässern des Alginats bei Raumtemperatur erfolgt.

11. Verfahren gemäß einem der vorhergehenden Ansprüche, bei dem als Algenmaterial in der Natur vorkommende Algenfrischmaterial oder in einem Bioreaktor bzw. Tankanlage kultiviertes Algenfrischmaterial oder Algenmaterial aus fusionierten oder regenerierten Algenzellen verwendet wird.

12. Verfahren gemäß einem der vorhergehenden Ansprüche, bei dem als Algenmaterial bestimmte Organ- oder Gewebeteile von Algen oder Algenteile bzw. bestimmte Organ- oder Gewebeteile von Algen oder Algenteilen aus bestimmten Stadien des Entwicklungszyklus von Algen verwendet werden.

13. Verfahren gemäß einem der vorhergehenden Ansprüche, bei dem als Algenmaterial Braunalgen oder andere alginatproduzierende Süßwasser- oder Salzwasseralgen verwendet werden.

14. Alginatzusammensetzung, hergestellt nach einem Verfahren nach einem der Ansprüche 1 bis 13,
**dadurch gekennzeichnet, daß** im Mischpolymer ein Verhältnis von Mannuronsäure zu Guluronsäure im Bereich von 1% bis 90% gegeben ist,
die Alginatzusammensetzung in wässriger Lösung mit einer 0.1%-igen Konzentration eine Viskosität im Bereich von 10 bis 15 mPa · s besitzt,
die Alginatzusammerisetzung in wässriger Lösung mit einer 0.5%-igen Konzentration eine Viskosität von 250 bis 300 mPa · s besitzt,
die Alginatzusammensetzung in wässriger Lösung bei Beleuchtung mit einer Anregungswellenlänge von 366 mm im Spektralbereich von 380 bis 550 nm keine Fluoreszenzemission zeigt,
die Alginatzusammensetzung bei Farbtests mit dem Folin-Denis-Reagenz oder mit Dimethoxybenzaldehyd keine Einfärbung zeigt,
die Alginatzusammensetzung in wässriger Lösung bei Beleuchtung mit einer Anregungswellenlänge von 270 nm im Spektralbereich von 300 bis 500 nm keine Fluoreszenzemission zeigt,
die Alginatzusammensetzung keine mit dem photometrischen Proteinnachweis nach Bradford nachweisbaren Proteine enthält,
die Alginatzusammensetzung bei Implantation in die Nieren von BB/OK-Ratten keine signifikante immunologische Reaktion auslöst und
die Alginatzusammensetzung nach Anwendung des XTT/MTT-Tests, oder der Zellrotationsmethode, oder einer elektrischen Zellzahl- und Zellgrößenbestimmung zu keiner nachweisbaren Lymphozytenaktivierung führt.

15. Verwendung einer Alginatzusammensetzung gemäß Anspruch 14 zur Herstellung von Alginatkapseln oder -umhüllungen für die Transplantationschirurgie und für andere medizinische Anwendungen und für die Lebensmittelindustrie.

16. Verwendung einer Alginatzusammensetzung gemäß Anspruch 14 zur Herstellung von Alginatkapseln oder -umhüllungen für allogene und xenogene Gewebe.

17. Verwendung einer Alginatzusammensetzung gemäß Anspruch 14 zur Herstellung von Alginatkapseln oder -umhüllungen für Langerhans'sche Inseln, Nebenschilddrüsengewebe, endokrines Gewebe oder Dopamin-sezernierende Zellen.

## Claims

1. Method for extracting a highly purified alginate composition from a mixed polymer made from mannuronic acid and guluronic acid, the average molecular weight of the mixed polymer being greater than 350 kD, comprising the steps:
a) extraction of algae material in a solution with a complexing agent,
b) sedimentation of cell components and particles with a porous binding agent,
c) filtration of the solution,
d) precipitation of alginate from the solution, and
e) collection and dehydration of the precipitated alginate, after which steps a) to e) are repeated at least once if so required, and
f) dissolving of the alginate and at least one further precipitation of the alginate.

2. Method according to claim 1 wherein ethylenediamene tetraacetic acid is used as a complexing agent for the extraction.

3. Method according to claim 1, wherein the extraction takes place in a soda solution.

4. Method according to claim 2 or 3, wherein activated carbon is added for the extraction of the solution.

5. Method according to claim 1, wherein the sedimentation takes place with a porous granulate based on diatomaceous earth, cellulose or recycling materials from renewable raw materials.

6. Method according to any of the preceding claims, wherein the filtration takes place with deep filters, each with a respectively smaller pore size.

7. Method according to any of the preceding claims, wherein the precipitation of alginate takes place with ethanol.

8. Method according to claim 7, wherein the ethanol content is selected in the range of 10-50 %.

9. Method according to any of the preceding claims, wherein the collection of the precipitated alginate takes place by foaming from the solution, by decanting the solution, or by stirring the solution with a stirring and collection device.

10. Method according to any of the preceding claims, wherein the alginate is dehydrated at ambient temperature.

11. Method according to any of the preceding claims, wherein fresh algae material as it occurs in nature, or fresh algae material cultivated in a bioreactor or a tank unit, or algae material from fused or regenerated algae cells is used as algae material.

12. Method according to any of the preceding claims, wherein specific organ or tissue parts of algae or algae parts or specific organ or tissue parts of algae or algae parts from specific stages of the development cycle of alge are used as algae material.

13. Method according to any of the preceding claims, wherein brown algae or other alginate-producing fresh water or salt water algae are used as algae material.

14. Alginate composition, produced according to a method according to any of claims 1 to 13, **characterised in that** in the mixed polymer there is a ratio of mannuronic acid to guluronic acid in the range of from 1 % to 90 %,
the alginate composition in the aqueous solution with a 0.1 % concentration has a viscosity in the range of from 10 to 15 mPa.s,
the alginate composition in the aqueous solution with a 0.5 % concentration has a viscosity of 250 to 300 mPa.s,
the alginate composition in the aqueous solution shows no fluorescence emission when illuminated with a stimulation wavelength of 366 mm in the spectral range of from 380 to 550 nm,
the alginate composition shows no colouring during colour tests with the Folin-Denis reagent or with dimethoxybenzaldehyde,
the alginate composition in the aqueous solution shows no fluorescence emission when illuminated with a stimulation wave length of 270 nm in the spectral range of from 300 to 500 nm,
the alignate composition does not contain any proteins detectable with the photometric protein evidence according to Bradford,
the alginate composition does not give rise to any significant immunological reaction when implanted in the kidneys of BB/OK rats, and
the alginate composition does not lead to any detectable lymphocyte activation following application of the XTT/MTT test or the cell rotation method or an electrical cell number and cell size determination.

15. Use of an alginate composition according to claim 14 in order to produce alginate capsules or coatings for transplantation surgery and for other medical appliations and for the food industry.

16. Use of an alginate composition according to claim 14 in order to produce alginate capsules or coatings for allogeneic and xenogenic tissues.

17. Use of an alginate composition according to claim 14 in order to produce alginate capsules or coatings for Langerhans' islands, parathyroid tissue, endocrine tissue or dopamine-secreting cells.

## Revendications

1. Procédé de production d'une composition d'alginate de haute pureté à base d'un copolymère d'acide mannuronique et d'acide guluronique, dans lequel le poids moléculaire moyen du copolymère est supérieur à 350 kD, comprenant les étapes consistant à :
a) extraire les substances d'algues en solution à l'aide d'un complexant,
b) faire sédimenter les constituants cellulaires et les particules à l'aide d'un liant poreux,
c) filtrer la solution,
d) précipiter l'alginate de la solution et
e) collecter et déshydrater l'alginate précipité puis répéter éventuellement au moins une fois les étapes a) à e) et
f) mettre l'alginate en solution et effectuer au moins une autre précipitation de l'alginate.

2. Procédé selon la revendication 1, dans lequel le complexant utilisé pour l'extraction consiste en de l'acide éthylène diamine tétraacétique.

3. Procédé selon la revendication 1, dans lequel l'extraction s'effectue dans une solution de soude.

4. Procédé selon la revendication 2 ou 3, dans lequel la solution est additionnée de charbon actif pour l'extraction.

5. Procédé selon la revendication 1, dans lequel la sédimentation s'effectue à l'aide de granulés poreux à base de kieselguhr, de cellulose ou de matières issues du recyclage de matières premières renouvelables.

6. Procédé selon l'une des revendications précédentes, dans lequel la filtration s'effectue à l'aide de filtres épais dont la taille des pores est chaque fois réduite.

7. Procédé selon l'une des revendications précédentes, dans lequel la précipitation de l'alginate s'effectue par l'éthanol.

8. Procédé selon la revendication 7, dans lequel la teneur en éthanol est sélectionnée dans la plage allant de 10 à 50 %.

9. Procédé selon l'une des revendications précédentes, dans lequel la collecte de l'alginate précipité s'effectue par moussage de la solution, décantation de la solution ou agitation de la solution à l'aide d'un dispositif d'agitation et de collecte.

10. Procédé selon l'une des revendications précédentes, dans lequel la déshydratation de l'alginate s'effectue à température ambiante.

11. Procédé selon l'une des revendications précédentes, dans lequel les substances d'algues utilisées consistent en des substances d'algues fraîches issues d'un milieu naturel ou bien de substances d'algues fraîches cultivées dans un bioréacteur ou un réservoir, ou bien en des substances d'algues issues de cellules d'algues fusionnées ou régénérées.

12. Procédé selon l'une des revendications précédentes, dans lequel les substances d'algues utilisées consistent en certaines parties d'organes ou de tissus d'algues ou en des parties d'algues, le cas échéant en certaines parties d'organes ou de tissus d'algues ou de parties d'algues se trouvant à certains stades du cycle de développement des algues.

13. Procédé selon l'une des revendications précédentes, dans lequel les substances d'algues utilisées consistent en des algues brunes ou d'autres algues d'eau douce ou d'eau salée produisant de l'alginate.

14. Composition d'alginate, préparée suivant un procédé selon l'une des revendications 1 à 13, **caractérisée en ce que**
- le copolymère présente un rapport entre acide mannuronique et acide guluronique situé dans la plage des 1 % à 90 %,
- la composition d'alginate en solution aqueuse possède une viscosité située dans la plage allant de 10 à 15 mPa·s avec une concentration de 0,1 %,
- la composition d'alginate en solution aqueuse possède une viscosité située dans la plage allant de 250 à 300 mPa·s avec une concentration de 0,5 %,
- la composition d'alginate en solution aqueuse ne présente aucune émission de fluorescence lorsqu'elle est soumise à un éclairage de longueur d'onde excitatrice de 366 nm dans la gamme spectrale comprise entre 380 et 550 nm,
- la composition d'alginate ne présente aucune coloration lors de tests de couleur au réactif de Folin-Denis ou au diméthoxybenzaldéhyde,
- la composition d'alginate en solution aqueuse ne présente aucune émission de fluorescence lorsqu'elle est soumise a un éclairage de longueur d'onde excitatrice de 270 nm dans la gamme spectrale comprise entre 300 et 500 nm,
- la composition d'alginate ne contient pas de protéines détectables selon le procédé de détection photométrique des protéines de Bradford,
- la composition d'alginate ne déclenche aucune réaction immunologique significative lorsqu'elle a été implantée dans les reins de rats BB/OK, et
- la composition d'alginate n'entraîne aucune activation démontrable des lymphocytes, par mise en évidence par le test XTT/MTT, par la méthode de rotation des cellules ou par détermination de la taille ou du nombre des cellules par un dispositif électrique.

15. Utilisation d'une composition d'alginate selon la revendication 14 pour la préparation de capsules ou d'enrobages à base d'alginate destinés à la chirurgie transplantatoire, à d'autres applications médicales et au secteur agro-alimentaire.

16. Utilisation d'une composition d'alginate selon la revendication 14 pour la préparation de capsules ou d'enrobages à base d'alginate destinés aux tissus allogènes et xénogènes.

17. Utilisation d'une composition d'alginate selon la revendication 14 pour la préparation de capsules ou d'enrobages à base d'alginate destinés aux îlots de Langerhans, aux tissus parathyroïdiens, aux tissus endocriniens ou aux cellules secrétant de la dopamine.
